# EUROPEAN PATENT APPLICATION

(11) **EP 3 854 353 A1**
(43) Date of publication of application: **28.07.2021**
(21) Application number: 19944797.0
(22) Date of filing: 11.09.2019
(51) Int. Cl.: A61F 2/38, A61F 2/30

(54) **KNEE JOINT IMPLANT PREVENTING HYPEREXTENSION**

(71) Applicant: Corentec Co., Ltd., Seoul 06649 (KR)
(72) Inventor: JANG, Young Woong, Seoul 06122 (KR); KIM, Chan Eol, Seoul 02801 (KR); RO, Jae Hun, Seoul 05571 (KR); HAN, Ah Reum, Chuncheon-si Gangwon-do 24318 (KR); LEE, Myung Chul, Seongnam-si Gyeonggi-do 13455 (KR); IN, Young, Seoul 06215 (KR); HAN, Seung Beom, Seoul 03010 (KR)
(74) Representative: Witte, Weller & Partner Patentanwälte mbB
(86) International application number: PCT/KR2019/011880
(87) International publication number: WO 2021/049689

(57) **Abstract**

The present disclosure relates to a knee joint implant for preventing hyper extension, and, more particularly, relates to a knee joint implant that includes a femur element of which the anterior surface has different radiuses of curvature in respective sections on the basis of the lowest point of a convex condyle and a bearing element of which the anterior surface has different radiuses of curvature in respective sections on the basis of the lowest point of a concave joint facet, wherein when an extended knee is about to be hyper-extended, the convex condyle of the femur element slides in the anterior direction to come into contact with the concave joint facet of the bearing element at a plurality of points so as to produce resistance, thereby suppressing hyper extension beyond design due to patient behavior, a decrease in the muscle mass of the patient, and the like.

## Description

### Technical Field

The present disclosure relates to a knee joint implant for preventing hyper extension, and, more particularly, relates to a knee joint implant that includes a femur element of which the anterior surface has different radiuses of curvature in respective sections on the basis of the lowest point of a convex condyle and a bearing element of which the anterior surface has different radiuses of curvature in respective sections on the basis of the lowest point of a concave joint facet, wherein when an extended knee is about to be hyper-extended, the convex condyle of the femur element slides in the anterior direction to come into contact with the concave joint facet of the bearing element at a plurality of points so as to produce resistance, thereby suppressing hyper extension beyond design due to patient behavior, a decrease in the muscle mass of the patient, and the like.

### Background Art

The knee joint indicates a joint including the three bones surrounding the knee, such as a femur, a tibia, and a patella, which is a core joint that supports the weight of a person and relates to exercises using the legs, such as walking or running through joint motion.

The femur has articular cartilage at the end thereof, and the tibia has a meniscus at the end thereof. If the cartilage is damaged due to extreme exercise or aging, the bones come into direct contact with each other, which may cause extreme pain.

As an orthopedic treatment method when such an injury occurs, total knee replacement (TKR) has been proposed to insert an implant capable of replacing the damaged knee joint into the patient's bone.

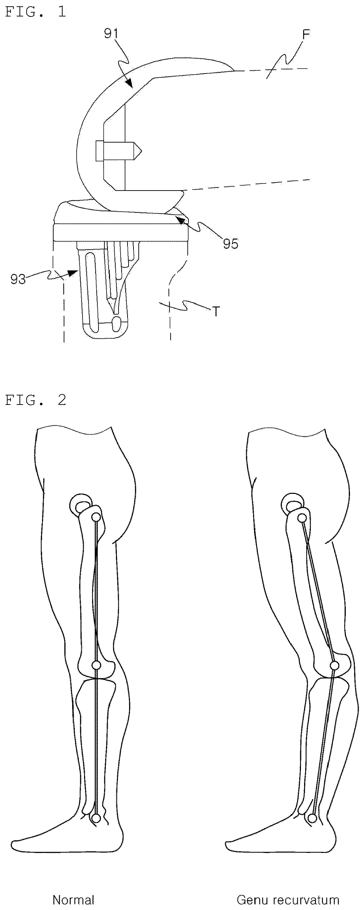

FIG. 1 is a diagram illustrating a conventional artificial knee joint disclosed in Korean Patent Publication No. 10-1184905 (20 SEP 2012). Hereinafter, a description will be made with reference to FIG. 1.

Total knee replacement is a surgery in which portions of a femur F and a tibia T are incised when the knee joint is damaged, and the incised portion is replaced with an artificial knee joint implant inserted thereinto. Implantation of the artificial knee joint implant is performed by a method of fixing a femur element 91 to a distal portion of the femur, fixing the tibia element 93 to a proximal portion of the tibia, and positioning a bearing element 95 between the femur element 91 and the tibia element 93.

However, one of the problems in the conventional total knee replacement is that the knee of the patient treated with total knee replacement is hyper-extended, thereby eventually causing genu recurvatum (back-knee) in which the knee joint is excessively bent back.

FIG. 2 is a diagram illustrating comparison between a normal knee and a back-knee. Referring to FIG. 2, the figure on the left side in FIG. 2 shows the appearance of a normal knee, and the figure on the right side in FIG. 2 shows the appearance of a back-knee in which the distal end of the femur and the proximal end of the tibia are excessively retracted in the posterior direction.

The genu recurvatum due to hyper extension after total knee replacement was mostly found in the patients having reduced muscle strength or poor walking habits, and, in particular, the genu recurvatum was found in elderly patients with severe aging of muscles, ligaments, and the like, so muscle weakness or the like may be a problem.

In addition, this problem stems from a problem in the shapes of the femur element and bearing element used in the conventional total knee replacement.

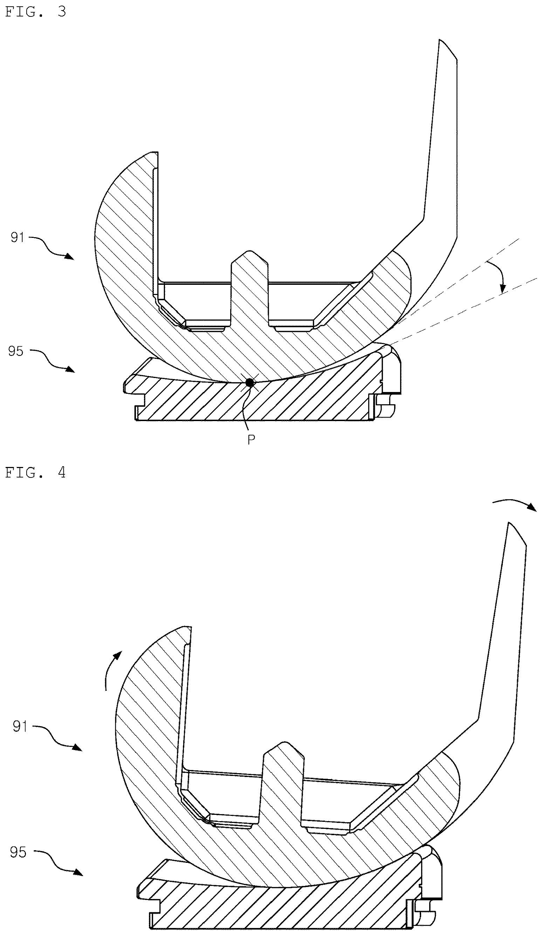

FIG. 3 is a diagram showing a one-point junction of the conventional femur element and bearing element in an extended state. As shown in FIG. 3, according to the shape of the conventional implant, a convex condyle of the femur element and a concave joint facet of the bearing element come into one-point contact at the sulcus point P, which indicates the lowest point of a curved surface, in an extended state.

The one-point contact at the sulcus point may cause the movement of the femur element that inclines in the anterior direction in the extended state.

Even with the one-point contact at the sulcus point, if the muscles, ligaments, and the like around the bone strongly hold the bone that is about to be out of position, the problem of hyper extension may not occur. However, in the case of the elderly patients having weak muscles, ligaments, and the like, since the strength of the muscles, ligaments, and the like is not sufficient, the knee is inevitably hyper-extended.

Consequently, the knee of an elderly patient treated with the total knee replacement is inclined to the area in which the condyle of the femur element comes into contact with the anterior joint facet of the bearing element as shown in FIG. 4, thereby bringing about genu recurvatum in which the knee is retracted back in an erect posture.

Therefore, the related industry is demanding the introduction of a new technology that prevents hyper extension through the structural shape of the implant itself even in the case of patients with weak muscles and the like, such as the elderly or the like.

(Patent Document 1) Korean Patent Publication No. 10-1184905 (20 SEP 2012)

### Detailed Description of the Invention

### Technical Problem

The present disclosure has been made in order to solve the above problems.

An objective of the present disclosure is to provide a knee joint implant configured such that a femur element having a convex condyle slides in the state in which the knee is extended to have a plurality of contact points with a bearing element having a concave joint facet, thereby preventing hyper extension even in the case where a patient who has aging muscles, ligaments, and the like is treated with total knee replacement.

Another objective of the present disclosure is to provide a knee joint implant including a femur element that has an approximately flat shape in a predetermined area from the lowest point of the convex condyle in an anterior direction such that the convex condyle of the femur element comes into two-point contact with the concave joint facet of the bearing element, thereby suppressing the occurrence of hyper extension of the knee, which is caused by the femur element that is inclined to the front of the contact point in the case of low muscle strength when the convex condyle of the femur element comes into one-point contact with the concave joint facet of the bearing element, through the structural shape of the implant even if muscle strength or the like does not act thereon.

Another objective of the present disclosure is to provide a knee joint implant configured such that the convex condyle of the femur element has a plurality of radiuses of curvature in the anterior direction of the lowest point and such that the concave joint facet of the bearing element corresponding thereto also has a plurality of radiuses of curvature in the anterior direction of the lowest point so that the condyle and the joint facet do not have sharply changing outer surfaces, thereby requiring more force than the implant having a simple radius of curvature for the occurrence of hyper extension.

Another objective of the present disclosure is to provide a knee joint implant having a special morphological feature in the area of the implant for joint motion so that when an anatomical movement crossing a predetermined boundary area is about to occur, resistance against the anatomical movement is generated by the morphological characteristics of the implant itself even if there is no assistance of surrounding muscles, ligaments, and the like.

Another objective of the present disclosure is to provide a knee joint implant in which the radius of curvature for each section of the condyle of the femur element is configured to be less than the radius of curvature for each section of the joint facet of the bearing element corresponding thereto even if the radiuses of curvature of the condyle of the femur element are different between the sections, thereby solving the problem with increased abrasion due to tight coupling with no extra tolerance when the radiuses of curvature are the same and providing stable flexibility in order to enable easy joint motion of the convex condyle of the femur element on the concave joint facet of the bearing element.

Another objective of the present disclosure is to provide a knee joint implant in which a second radius of curvature of a second section of the femur element is configured to be less than a first radius of curvature of a first section thereof, thereby reflecting a typical anatomical shape of the patient to the second section of the femur element.

Another objective of the present disclosure is to provide a knee joint implant in which a third radius of curvature of a third section of the femur element is configured to be less than a first radius of curvature of a first section and greater than a second radius of curvature of a second section so that the condyle of the femur element slides on the bearing element and then comes into two-point contact therewith when an extended knee is about to be hyper-extended, thereby preventing further hyper extension.

Another objective of the present disclosure is to provide a knee joint implant in which a first angle of the condyle of the femur element is configured to be less than a second angle thereof to minimize the section in which the condyle of the femur element slides on the joint facet of the bearing element, thereby preventing unnatural motion of the patient.

Another objective of the present disclosure is to provide a knee joint implant in which the bearing element has an approximately flat shape in a predetermined area from the lowest point of the concave joint facet in the anterior direction so that the approximately flat first section of the femur element is placed thereon and so that, when an extended knee is about to be hyper-extended, the first section of the femur element slides on a fourth section of the bearing element and then comes into two-point contact therewith, thereby preventing hyper extension.

Another objective of the present disclosure is to provide a knee joint implant in which a fifth radius of curvature of a fifth section of the bearing element is configured to be less than a fourth radius of curvature of the fourth section, thereby reflecting a typical anatomical shape of the patient to the fifth section of the bearing element.

Another objective of the present disclosure is to provide a knee joint implant in which a sixth radius of curvature of a sixth section of the bearing element is configured to be less than a fourth radius of curvature of a fourth section and greater than a fifth radius of curvature of a fifth section so that the condyle of the femur element slides on the bearing element and then comes into two-point contact therewith when an extended knee is about to be hyper-extended, thereby preventing further hyper extension.

Another objective of the present disclosure is to provide a knee joint implant in which a fourth angle of the bearing element is configured to be less than a fifth angle thereof to minimize the section in which the condyle of the femur element slides on the joint facet of the bearing element, thereby preventing unnatural motion of the patient.

Another objective of the present disclosure is to provide a knee joint implant in which the area of a first section is configured to be less than the area of a fourth section so that the first section of the femur element is able to stably slide on the fourth section of the bearing element.

### Technical Solution

The present disclosure is implemented by the embodiments having the following configurations in order to attain the above objectives.

According to an embodiment of the present disclosure, the present disclosure is characterized by including a femur element in which an anterior surface has different radiuses of curvature in respective sections on the basis of the lowest point of a convex condyle, and when an extended knee is about to be hyper-extended, the condyle of the femur element slides in an anterior direction along a concave joint facet of a bearing element, and then comes into contact with the joint facet at a plurality of points.

According to another embodiment of the present disclosure, the present disclosure is characterized in that the femur element includes a first section having a first radius of curvature from the lowest point of the condyle and extending in the anterior direction at a first angle from the center of the first radius of curvature.

According to another embodiment of the present disclosure, the present disclosure is characterized in that the femur element includes a second section having a second radius of curvature from an end of the first section and extending in the anterior direction at a second angle from the center of the second radius of curvature and a third section having a third radius of curvature from an end of the second section and extending in the anterior direction at a third angle from the center of the third radius of curvature, and points that come into contact with the joint facet when the extended knee is about to be hyper-extended are formed in the first section and the third section.

According to another embodiment of the present disclosure, the present disclosure is characterized in that the first radius of curvature is greater than the second radius of curvature.

According to another embodiment of the present disclosure, the present disclosure is characterized in that the third radius of curvature is less than the first radius of curvature and greater than the second radius of curvature.

According to another embodiment of the present disclosure, the present disclosure is characterized in that the first angle is less than the second angle.

According to another embodiment of the present disclosure, the present disclosure is characterized by including a bearing element in which an anterior surface has different radiuses of curvature in respective sections on the basis of the lowest point of a concave joint facet, and when an extended knee is about to be hyper-extended, the bearing element comes into contact with a condyle of a femur element, which slides in an anterior direction along the joint facet, at a plurality of points.

According to another embodiment of the present disclosure, the present disclosure is characterized in that the bearing element includes a fourth section having a fourth radius of curvature from the lowest point of the joint facet and extending in the anterior direction at a fourth angle from the center of the fourth radius of curvature.

According to another embodiment of the present disclosure, the present disclosure is characterized in that the bearing element includes a fifth section having a fifth radius of curvature from an end of the fourth section and extending in the anterior direction at a fifth angle from the center of the fifth radius of curvature and a sixth section having a sixth radius of curvature from an end of the fifth section and extending in the anterior direction at a sixth angle from the center of the sixth radius of curvature, and points that come into contact with the condyle when the extended knee is about to be hyper-extended are formed in the fourth section and the sixth section.

According to another embodiment of the present disclosure, the present disclosure is characterized in that the fourth radius of curvature is greater than the fifth radius of curvature.

According to another embodiment of the present disclosure, the present disclosure is characterized in that the sixth radius of curvature is less than the fourth radius of curvature and greater than the fifth radius of curvature.

According to another embodiment of the present disclosure, the present disclosure is characterized in that the fourth angle is less than the fifth angle.

According to another embodiment of the present disclosure, the present disclosure is characterized by including a femur element in which an anterior surface has different radiuses of curvature in respective sections on the basis of the lowest point of a convex condyle and a bearing element in which an anterior surface has different radiuses of curvature in respective sections on the basis of the lowest point of a concave joint facet, and when an extended knee is about to be hyper-extended, the condyle of the femur element slides in an anterior direction along the joint facet of the bearing element, and then comes into contact with the joint facet at a plurality of points.

According to another embodiment of the present disclosure, the present disclosure is characterized in that the femur element includes a first section having a first radius of curvature from the lowest point of the condyle and extending in the anterior direction at a first angle from the center of the first radius of curvature, and wherein the bearing element includes a fourth section having a fourth radius of curvature from the lowest point of the joint facet and extending in the anterior direction at a fourth angle from the center of the fourth radius of curvature.

According to another embodiment of the present disclosure, the present disclosure is characterized in that the femur element includes a second section having a second radius of curvature from an end of the first section and extending in the anterior direction at a second angle from the center of the second radius of curvature, and a third section having a third radius of curvature from an end of the second section and extending in the anterior direction at a third angle from the center of the third radius of curvature, and the bearing element includes a fifth section having a fifth radius of curvature from an end of the fourth section and extending in the anterior direction at a fifth angle from the center of the fifth radius of curvature, and a sixth section having a sixth radius of curvature from an end of the fifth section and extending in the anterior direction at a sixth angle from the center of the sixth radius of curvature, and when the extended knee is about to be hyper-extended, the first section and the fourth section, and the third section and the sixth section form contact points, respectively.

According to another embodiment of the present disclosure, the present disclosure is characterized in that the first radius of curvature is less than the fourth radius of curvature, the second radius of curvature is less than the fifth radius of curvature, and the third radius of curvature is less than the sixth radius of curvature.

According to another embodiment of the present disclosure, the present disclosure is characterized in that an area of the first section is less than an area of the fourth section.

### Advantageous Effects

The present disclosure is able to obtain the following effects by the present embodiment and the configurations, combinations, and a usage relationship, which will be described below.

The present disclosure has an effect of providing a knee joint implant configured such that a femur element having a convex condyle slides in the state in which the knee is extended to have a plurality of contact points with a bearing element having a concave joint facet, thereby preventing hyper extension even in the case where a patient who has aging muscles, ligaments, and the like is treated with total knee replacement.

The present disclosure has an effect of providing a knee joint implant including a femur element that has an approximately flat shape in a predetermined area from the lowest point of the convex condyle in an anterior direction such that the convex condyle of the femur element comes into two-point contact with the concave joint facet of the bearing element, thereby suppressing the occurrence of hyper extension of the knee, which is caused by the femur element that is inclined to the front of the contact point in the case of low muscle strength when the convex condyle of the femur element comes into one-point contact with the concave joint facet of the bearing element, through the structural shape of the implant even if muscle strength or the like does not act thereon.

The present disclosure has an effect of providing a knee joint implant configured such that the convex condyle of the femur element has a plurality of radiuses of curvature in the anterior direction of the lowest point and such that the concave joint facet of the bearing element corresponding thereto also has a plurality of radiuses of curvature in the anterior direction of the lowest point so that the condyle and the joint facet do not have sharply changing outer surfaces, thereby requiring more force than the implant having a simple radius of curvature for the occurrence of hyper extension.

The present disclosure has an effect of providing a knee joint implant having a special morphological feature in the area of the implant for joint motion so that when an anatomical movement crossing a predetermined boundary area is about to occur, resistance against the anatomical movement is generated by the morphological characteristics of the implant itself even if there is no assistance of surrounding muscles, ligaments, and the like.

The present disclosure has an effect of providing a knee joint implant in which the radius of curvature for each section of the condyle of the femur element is configured to be less than the radius of curvature for each section of the joint facet of the bearing element corresponding thereto even if the radiuses of curvature of the condyle of the femur element are different between the sections, thereby solving the problem with increased abrasion due to tight coupling with no extra tolerance when the radiuses of curvature are the same and providing stable flexibility in order to enable easy joint motion of the convex condyle of the femur element on the concave joint facet of the bearing element.

The present disclosure has an effect of providing a knee joint implant in which a second radius of curvature of a second section of the femur element is configured to be less than a first radius of curvature of a first section thereof, thereby reflecting a typical anatomical shape of the patient to the second section of the femur element.

The present disclosure has an effect of providing a knee joint implant in which a third radius of curvature of a third section of the femur element is configured to be less than a first radius of curvature of a first section and greater than a second radius of curvature of a second section so that the condyle of the femur element slides on the bearing element and then comes into two-point contact therewith when an extended knee is about to be hyper-extended, thereby preventing further hyper extension.

The present disclosure has an effect of providing a knee joint implant in which a first angle of the condyle of the femur element is configured to be smaller than a second angle thereof to minimize the section in which the condyle of the femur element slides on the joint facet of the bearing element, thereby preventing unnatural motion of the patient.

The present disclosure has an effect of providing a knee joint implant in which the bearing element has an approximately flat shape in a predetermined area from the lowest point of the concave joint facet in the anterior direction so that the approximately flat first section of the femur element is placed thereon and so that, when an extended knee is about to be hyper-extended, the first section of the femur element slides on a fourth section of the bearing element and then comes into two-point contact therewith, thereby preventing hyper extension.

The present disclosure has an effect of providing a knee joint implant in which a fifth radius of curvature of a fifth section of the bearing element is configured to be less than a fourth radius of curvature of the fourth section, thereby reflecting a typical anatomical shape of the patient to the fifth section of the bearing element.

The present disclosure has an effect of providing a knee joint implant in which a sixth radius of curvature of a sixth section of the bearing element is configured to be less than a fourth radius of curvature of a fourth section and greater than a fifth radius of curvature of a fifth section so that the condyle of the femur element slides on the bearing element and then comes into two-point contact therewith when an extended knee is about to be hyper-extended, thereby preventing further hyper extension.

The present disclosure has an effect of providing a knee joint implant in which a fourth angle of the bearing element is configured to be less than a fifth angle thereof to minimize the section in which the condyle of the femur element slides on the joint facet of the bearing element, thereby preventing unnatural motion of the patient.

The present disclosure has an effect of providing a knee joint implant in which the area of a first section is configured to be less than the area of a fourth section so that the first section of the femur element is able to stably slide on the fourth section of the bearing element.

### Brief Description of the Drawings

FIG. 1 is a diagram illustrating a conventional artificial knee joint.
FIG. 2 is a diagram illustrating comparison between a normal knee and genu recurvatum.
FIG. 3 is a diagram illustrating one-point junction of the conventional femur element and bearing element in an extended state.
FIG. 4 is a diagram illustrating the state in which the implant in FIG. 3 is hyper-extended.
FIG. 5 is a perspective view of a knee joint implant according to an embodiment of the present disclosure.
FIG. 6 is an exploded perspective view of FIG. 5.
FIG. 7 is a perspective view of a femur element according to an embodiment of the present disclosure.
FIG. 8 is a cross-sectional view taken along the line A-A' in FIG. 7.
FIG. 9 is a diagram illustrating radiuses of curvature and angles in respective sections in FIG. 8.
FIG. 10 is a perspective view of a bearing element according to an embodiment of the present disclosure.
FIG. 11 is a cross-sectional view taken along the line B-B' in FIG. 10.
FIG. 12 is a diagram illustrating radiuses of curvature and angles in the respective sections in FIG. 11.
FIG. 13 is a diagram illustrating contact points of a knee joint implant in an extended state according to an embodiment of the present disclosure.
FIG. 14 is a diagram illustrating contact points for preventing hyper extension of a knee joint implant according to an embodiment of the present disclosure.

### Mode for Carrying Out the Invention

Hereinafter, preferred embodiments of a knee joint implant preventing hyper extension according to the present disclosure will be described in detail with reference to the accompanying drawings. In the following description of the present disclosure, if it is determined that a detailed description of a known function or configuration may unnecessarily obscure the subject matter of the present disclosure, the detailed description thereof will be omitted. Unless otherwise defined, all terms in the present specification are the same as the general meaning of the terms understood by those skilled in the art to which the present disclosure pertains, and if a term conflicts with the meaning of the term used in the present specification, the term will follow the definition used in the present specification.

In this specification, it is noted that the drawings will show a knee joint implant to be inserted into the left knee of a patient.

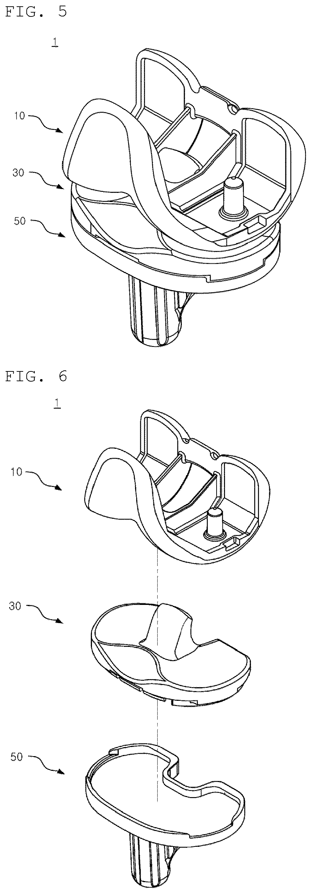

FIG. 5 is a perspective view of a knee joint implant according to an embodiment of the present disclosure, and FIG. 6 is an exploded perspective view of FIG. 5. Referring to FIGS. 5 and 6, a knee joint implant 1 for preventing hyper extension according to the present disclosure is an implant used in the surgery for total knee replacement, and may be preferably inserted between a distal portion of a femur and a proximal portion of a tibia, and between the distal portion of the femur and the proximal portion of the tibia. Specifically, as shown in FIGS. 5 and 6, the knee joint implant includes a femur element 10, a bearing element 30, and a tibia element 50.

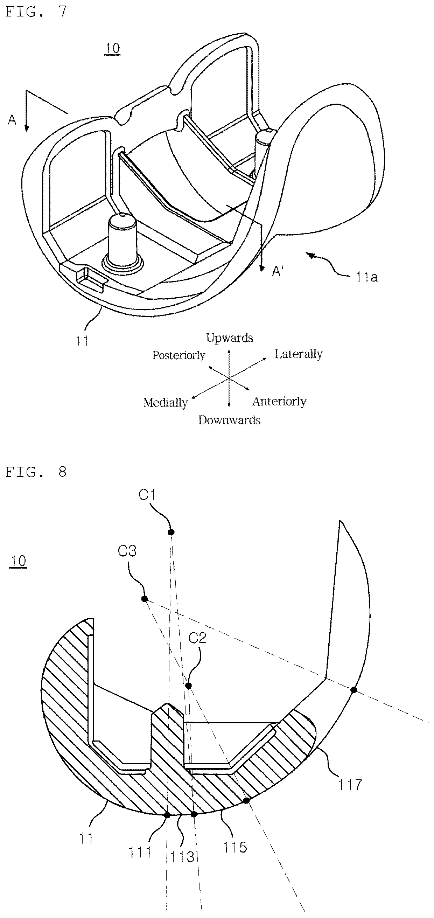

FIG. 7 is a perspective view of a femur element according to an embodiment of the present disclosure. Referring to FIG. 7, the femur element 10 is an implant that is inserted into the distal portion of the femur after cutting out a predetermined portion of a convex distal end of the femur in order to replace the cut portion. The femur element 10 may be configured such that an anterior surface 11a thereof has different radiuses of curvature in respective sections on the basis of the lowest point 111 of a convex condyle 11, which will be described later. Thanks to the different radiuses of curvature, even if an elderly patient, having aging muscles, ligaments, and the like, is treated with total knee replacement, when an extended knee is about to be hyper-extended due to the act of going down stairs or the like, a condyle 11, which will be described later, may slide in the anterior direction along a concave joint facet 31 of the bearing element 30, which will be described later, to then come into contact with the joint facet 31 at a plurality of points, thereby preventing a hyper extension phenomenon through the structural shape of the implant. The femur element 10 includes a condyle 11.

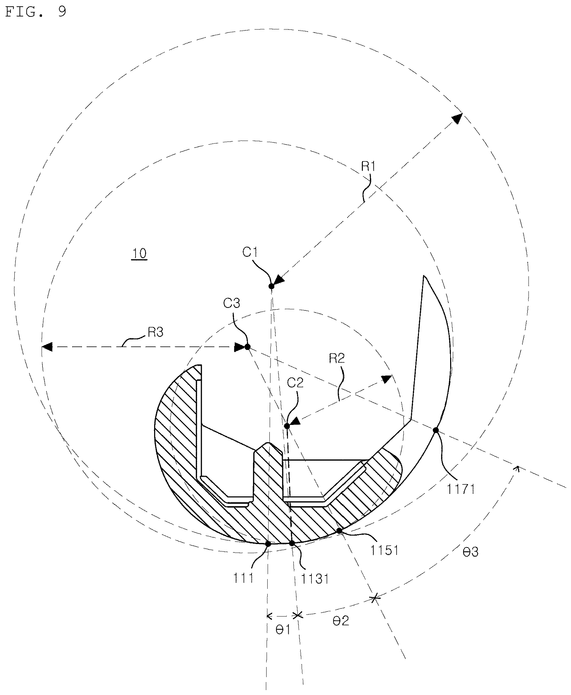

The condyle 11 is a portion configured to be convex downwards in the femur element 10 that is divided into a medial side and a lateral side, as shown in FIG. 7, and comes into contact with the joint facet of the bearing element 30, which will be described later, thereby enabling joint motion. FIG. 8 is a cross-sectional view taken along the line A-A' in FIG. 7, and FIG. 9 is a diagram illustrating radiuses of curvature and angles in the respective sections in FIG. 8. Referring to FIGS. 8 and 9, the condyle 11 includes a lowest point 111, a first section 113, a second section 115, and a third section 117.

The lowest point 111 indicates the point located in the lowest portion on the curved surface of the femur element 10, and, as shown in FIGS. 8 and 9, is used to indicate the lowest point of the curved surface that is convex at the condyle 11 of the femur element 10, which is configured to be convex downwards. In an extended state in which the patient stands upright, the lowest point 111 of the femur element 10 may be configured to come into contact with the lowest point 311 of the joint facet 31 of the bearing element 30, which will be described later. The surface of the condyle 11 in the anterior direction on the basis of the lowest point 111 will be referred to as an "anterior surface" 11a, and the present disclosure is characterized in that the anterior surface 11a has various radiuses of curvature.

The first section 113, as shown in FIGS. 8 and 9, has a first radius of curvature R1 from the lowest point 111 of the condyle 11, and extends in the anterior direction at a first angle θ1 from the center C1 of the first radius of curvature. Preferably, the first section 113 may be configured to be generally horizontal.

The first section 113 is a portion formed to be approximately horizontal on the condyle 11 having a convex shape as a whole, and allows the femur element 10 to slide on the joint facet 31 of the bearing element 30, which will be described later. Sliding means that the femur element 10 slides in the anterior direction on the joint facet 31 of the bearing element 30, which will be described later, and sliding is not to be limitedly interpreted only as translational motion of the femur element 10, but may be preferably interpreted as translational motion involving predetermined rotational motion.

As described above, since the first section 113 induces the sliding of the femur element 10 on the bearing element 30, if the first section 113 is excessively long, the patient's motion may be unnatural. Thus, it may be desirable to minimize the length of the first section 113. Therefore, more preferably, the first angle θ1 may be configured to be less than a second angle θ2 (θ1<θ2), which will be described later.

As will be described in detail later, the present disclosure configures two contact points in order to prevent the extended knee from being hyper-extended due to the act of going down stairs or the like, and one of the contact points is formed in the first section 113.

The first radius of curvature R1 and the first angle θ1 of the first section 113 are not limited to any specific concept, and preferably, the first radius of curvature R1 may be 85 mm, and the first angle θ1 may be 11 degrees.

As shown in FIGS. 8 and 9, the second section 115 has a second radius of curvature R2 from the end 1131 of the first section 113, and extends in the anterior direction at a second angle θ2 from the center C2 of the second radius of curvature.

The second section 115 is a portion corresponding to a typical anatomical shape of the patient, and the first section 113 is a substantially horizontal section, whereas the second section 115 may have a curvature, which changes somewhat sharply because it implements the typical anatomical shape of the patient. Preferably, the second radius of curvature R2 may be configured to be less than the first radius of curvature R1 (R1>R2) such that the second section 115 does not come into contact with the joint facet 31 of the bearing element 30, which will be described later, when an extended knee is about to be hyper-extended.

In addition, if the first section 113 for inducing the sliding of the femur element 10 is lengthened, the patient's motion becomes unnatural. Thus, since the first section 113 is preferably minimized, the second angle θ2 may be configured to be greater than the first angle θ1 (θ2>θ1).

The second radius of curvature R2 and the second angle θ2 of the second section 115 are not limited to any specific concept, and preferably, the second radius of curvature R2 may be 26.5 mm, and the second angle θ2 may be 14 degrees.

The third section 117 has a third radius of curvature R3 from the end 1151 of the second section 115, and extends to the end 1171 in the anterior direction from the center C3 of the third radius of curvature at a third angle θ3. A section having another radius of curvature is configured beyond the end 1171 of the third section 117, and the section comes into contact with a patella implant (not shown). A description of the section beyond the end 1171 of the third section 117 will be omitted.

In order to prevent an extended knee from being hyper-extended due to the act of going down stairs or the like, the present disclosure preferably configures two contact points, wherein one of the contact points may be formed in the first section 113 and the remaining one thereof may be formed in the third section 117. To this end, preferably, the third radius of curvature R3 may be configured to be less than the first radius of curvature R1 and greater than the second radius of curvature R2 (R1>R3>R2).

The third radius of curvature R3 of the third section 117 is not limited to a specific concept, and preferably, the third radius of curvature R3 may be 38.5 mm.

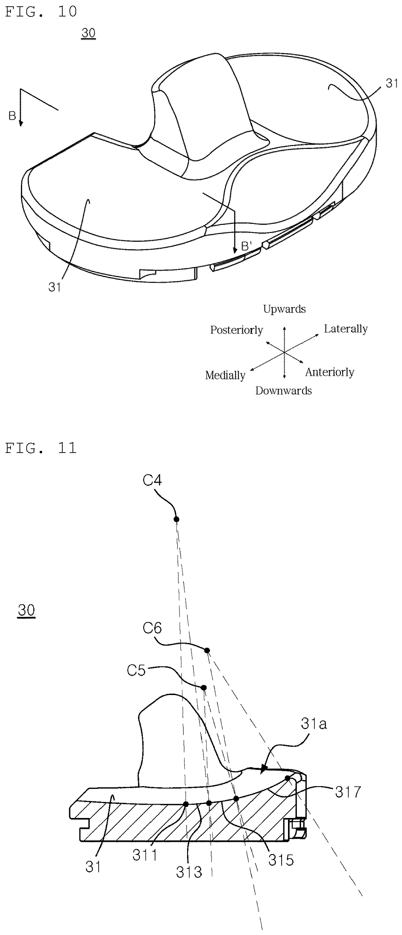

FIG. 10 is a perspective view of a bearing element according to an embodiment of the present disclosure. Referring to FIG. 10, a bearing element 30 is configured to be placed on the tibia element 50, which is inserted into the proximal portion of the tibia after cutting out a predetermined portion of the proximal end of the tibia in order to replace the cut portion, and come into contact with the condyle 11 of the femur element 10. The tibia element 30 may be configured such that the anterior surface 31a has different radiuses of curvature in respective sections on the basis of the lowest point 311 of the concave joint facet 31, which will be described later. Thanks to the different radiuses of curvature, even if an elderly patient, having aging muscles, ligaments, or the like, is treated with total knee replacement, when an extended knee is about to be hyper-extended due to the act of going down stairs or the like, the condyle 11 may slide in the anterior direction along the concave joint facet 31 of the bearing element 30 to then come into contact with the joint facet 31 at a plurality of points, thereby preventing a hyper extension phenomenon through the structural shape of the implant. The tibia element 30 includes the joint facet 31.

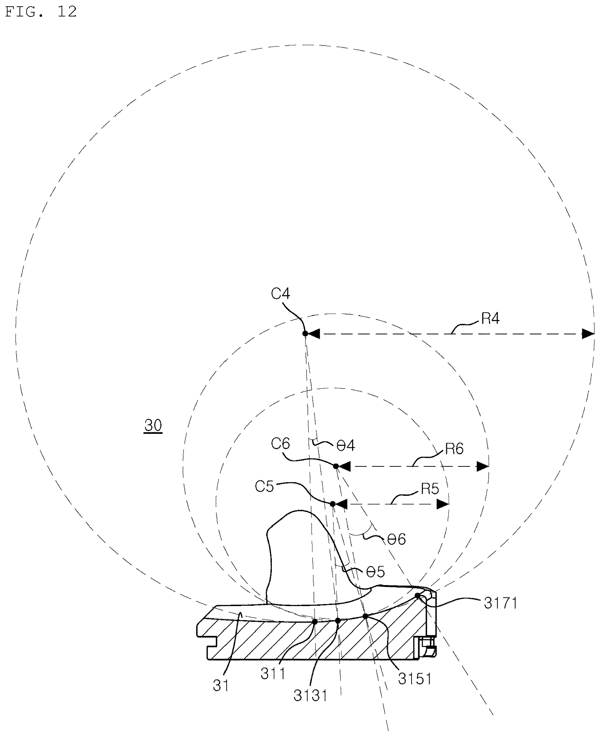

The joint facet 31 is a portion configured to be recessed downwards in the bearing element 30 divided into a medial side and a lateral side, as shown in FIG. 10. The joint facet comes into contact with the convex condyle 11 of the femur element 10, thereby enabling joint motion. FIG. 11 is a cross-sectional view taken along the line B-B' in FIG. 10, and FIG. 12 is a diagram illustrating radiuses of curvature and angles in the respective sections in FIG. 11. Referring to FIGS. 11 and 12, the joint facet 31 includes a lowest point 311, a fourth section 313, a fifth section 315, and a sixth section 317.

The lowest point 311 indicates the most recessed point on the curved surface of the concave joint facet 31 of the bearing element 30, and, as shown in FIGS. 11 and 12, is used to indicate the lowest point of the concave curved surface in the joint facet 31 of the bearing element 30, which is recessed downwards. Preferably, the aforementioned lowest point 111 of the femur element 10 may be configured to come into contact with the lowest point 311 of the bearing element 30 in an extended state in which the patient stands upright. The surface of the joint facet 31 in the anterior direction on the basis of the lowest point 311 will be referred to as an "anterior surface" 31a, and the present disclosure is characterized in that the anterior surface 31a has various radiuses of curvature.

The fourth section 313 is a portion that has a fourth radius of curvature R4 from the lowest point 311 of the joint facet 31 and extends in the anterior direction at a fourth angle θ4 from the center C4 of the fourth radius of curvature. Preferably, the fourth section 313 may be configured to be generally horizontal. Since the fourth section 313 is a portion on which the first section 113, which is configured to be generally horizontal, is placed, the area of the fourth section 313 is preferably configured to be greater than the area of the first section 113.

A point, which comes into contact with the condyle 11 of the femur element 10 when an extended knee is about to be hyper-extended, may be formed in the fourth section 313, and preferably, the first section 113 of the condyle 11 and the fourth section 313 may form contact points P.

In order for the condyle 11 of the femur element 10 to perform smooth joint motion while being placed on the joint facet 31 of the bearing element 30, the curvatures of the respective sections of the condyle 11 are preferably configured to be less than the curvatures of the respective sections of the joint facet 31. If the curvatures of the respective sections of the joint facet 31 are the same as the curvatures of the respective sections of the condyle 11, there is no extra tolerance, which brings tight contact between the condyle 11 and the joint facet 31, thereby accelerating abrasion of the implant. Consequently, the curvatures of the joint facet 31 of the bearing element are preferably configured to be greater than the curvatures of the respective sections of the condyle 11 corresponding thereto. Accordingly, the fourth radius of curvature R4 may be configured to be greater than the first radius of curvature R1.

The fourth radius of curvature R4 and the fourth angle θ4 of the fourth section 313 are not limited to any specific concept, and preferably, the fourth radius of curvature R4 may be 85.5 mm, and the fourth angle θ4 may be 5 degrees.

The fifth section 315 is a portion that has a fifth radius of curvature R5 from the end 3131 of the fourth section 313 and extends in the anterior direction at a fifth angle θ5 from the center C5 of the fifth radius of curvature.

The fifth section 315 is a portion corresponding to a typical anatomical shape of the patient, and the fourth section 313 is a substantially horizontal section, whereas the fifth section 115 may have a curvature, which may change somewhat sharply, because it implements the typical anatomical shape of the patient. Preferably, the fifth radius of curvature R5 may be configured to be less than the fourth radius of curvature R4 (R4>R5) such that the fifth section 315 does not come into contact with the condyle 11 when an extended knee is about to be hyper-extended.

The fifth section 315 is a portion corresponding to the second section 115 of the condyle 11, and as described above, the fifth radius of curvature R5 of the fifth section 315 is preferably configured to be greater than the second radius of curvature R2 of the second section 115 so as to enable smooth joint motion of the condyle 10 of the femur element 10 on the joint facet 31 of the bearing element 30.

The fifth section 315 supports the sliding of the first section 113 of the femur element 10, which is generally horizontal, and if the sliding section is long, there is a problem in which the patient's motion becomes unnatural. Thus, since the fifth section 315 is preferably minimized, the fifth angle θ5 may be configured to be greater than the fourth angle θ4 (θ5>θ4).

The fifth radius of curvature R5 and the fifth angle θ5 of the fifth section 315 are not limited to any specific concept, and preferably, the fifth radius of curvature R5 may be 27 mm, and the fifth angle θ5 may be 9 degrees.

The sixth section 317 is a portion that has a sixth radius of curvature R6 from the end 3151 of the fifth section 315 and extends in the anterior direction at a sixth angle θ6 from the center C6 of the sixth radius of curvature. In order to prevent an extended knee from being hyper-extended due to the act of going down stairs or the like, the present disclosure preferably configures two contact points, wherein one of the contact points may be formed in the fourth section 313 and the remaining one thereof may be formed in the sixth section 317. To this end, preferably, the sixth radius of curvature R6 may be configured to be less than the fourth radius of curvature R4 and greater than the fifth radius of curvature R5 (R4>R6>R5).

The first section 113 of the condyle 11 and the fourth section 313 of the joint facet 31, and the third section 117 of the condyle 11 and the sixth section 317 of the joint facet 31 form contact points P, respectively, and thus the knee joint implant 1 of the present disclosure forms a plurality of contact points P when an extended knee is about to be hyper-extended by the act of stretching the knee while going down stairs or the like, thereby preventing hyper extension of the joint.

The sixth section 317 is a portion corresponding to the third section 117 of the condyle 11, and the sixth radius of curvature R6 of the sixth section 317 is preferably configured to be greater than the third radius of curvature R3 of the third section 117 so as to enable smooth joint motion of the condyle 10 of the femur element 10 on the joint facet 31 of the bearing element 30.

The sixth radius of curvature R6 of the sixth section 317 is not limited to any specific concept, and preferably, the sixth radius of curvature R6 may be 39 mm.

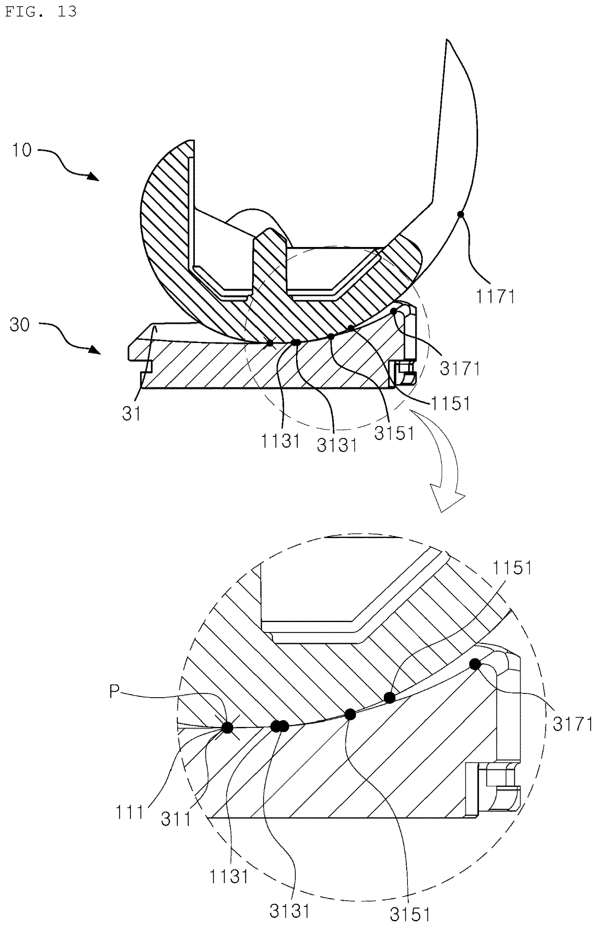

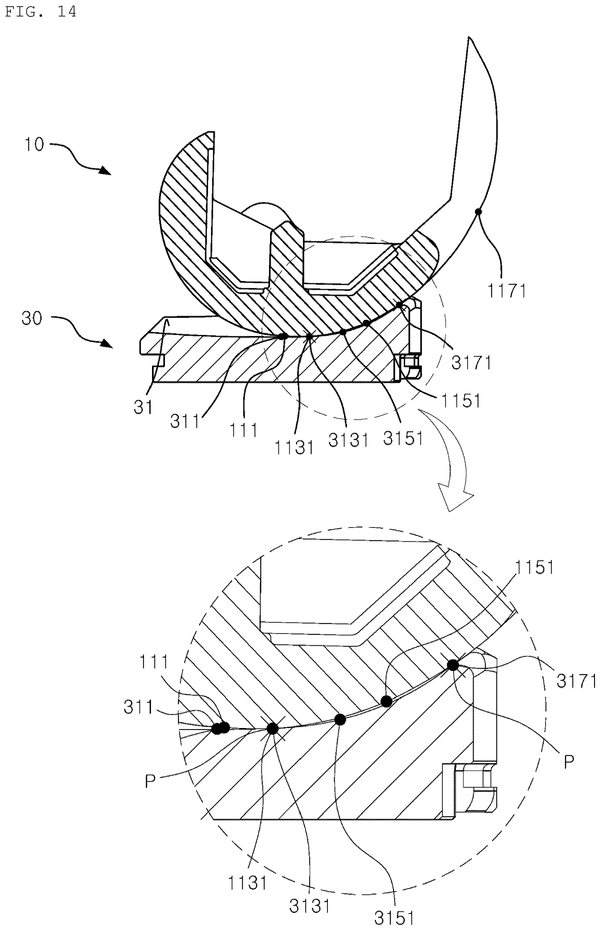

FIG. 13 is a diagram illustrating contact points of a knee joint implant in an extended state according to an embodiment of the present disclosure, and FIG. 14 is a diagram illustrating contact points for preventing hyper extension of a knee joint implant according to an embodiment of the present disclosure. Hereinafter, a description will be made with reference to FIGS. 13 and 14.

When a patient stands upright, the knee is in an extended state, and at this time, the lowest point 111 of the femur element 10 comes into one-point contact P corresponding to the lowest point 311 of the bearing element 30 as shown in FIG. 13.

In this situation, when the patient who was treated with total knee replacement performs an act such as raising a foot off the floor to go down stairs or the like, the extended knee is about to be hyper-extended.

At this time, the femur element 10 slides on the bearing element 30, and this may be performed by the first section 113 and the fourth section 313, which are generally horizontal.

When the femur element 10 slides, as shown in FIG. 14, the lowest point 111 of the femur element 10 moves in the anterior direction of the lowest point 311 of the bearing element 30. The end 1131 of the first section 113 may form a contact point P with the end 3131 of the fourth section 313, and at this time, the third section 117 of the femur element 10 may form another contact point P with the end 3171 of the sixth section 317 of the bearing element 30, whereby the condyle 11 of the element 10 and the joint facet 31 of the bearing element 30 come into two-point contact P when the extended knee is about to be hyper-extended, thereby preventing further hyper extension.

The detailed description above is illustrative of the present disclosure. In addition, the above description shows and describes preferred embodiments of the present disclosure, and the present disclosure may be used in various other combinations, modifications, and environments. That is, changes or modifications may be made within the scope of the concept of the present disclosure disclosed in the present specification, the scope equivalent to the disclosed content, and/or the skill or knowledge of the art. The above-described embodiments provide the best mode for implementing the technical idea of the present disclosure, and various changes, required in the specific application fields and usage of the present disclosure, are possible. Therefore, the detailed description of the present disclosure is not intended to limit the present disclosure to the disclosed embodiments. In addition, the appended claims should be construed as including other embodiments.

## Claims

1. A knee joint implant for preventing hyper extension, the knee joint implant comprising a femur element in which an anterior surface has different radiuses of curvature in respective sections on the basis of the lowest point of a convex condyle,
wherein when an extended knee is about to be hyper-extended, the condyle of the femur element slides in an anterior direction along a concave joint facet of a bearing element, and then comes into contact with the joint facet at a plurality of points.

2. The knee joint implant for preventing hyper extension of claim 1, wherein the femur element comprises a first section having a first radius of curvature from the lowest point of the condyle and extending in the anterior direction at a first angle from the center of the first radius of curvature.

3. The knee joint implant for preventing hyper extension of claim 2, wherein the femur element comprises:
a second section having a second radius of curvature from an end of the first section and extending in the anterior direction at a second angle from the center of the second radius of curvature; and
a third section having a third radius of curvature from an end of the second section and extending in the anterior direction at a third angle from the center of the third radius of curvature, and
wherein points that come into contact with the joint facet when the extended knee is about to be hyper-extended are formed in the first section and the third section.

4. The knee joint implant for preventing hyper extension of claim 3, wherein the first radius of curvature is greater than the second radius of curvature.

5. The knee joint implant for preventing hyper extension of claim 4, wherein the third radius of curvature is less than the first radius of curvature and greater than the second radius of curvature.

6. The knee joint implant for preventing hyper extension of claim 3, wherein the first angle is less than the second angle.

7. A knee joint implant for preventing hyper extension, the knee joint implant comprising a bearing element in which an anterior surface has different radiuses of curvature in respective sections on the basis of the lowest point of a concave joint facet,
wherein when an extended knee is about to be hyper-extended, the bearing element comes into contact with a condyle of a femur element, which slides in an anterior direction along the joint facet, at a plurality of points.

8. The knee joint implant for preventing hyper extension of claim 7, wherein the bearing element comprises a fourth section having a fourth radius of curvature from the lowest point of the joint facet and extending in the anterior direction at a fourth angle from the center of the fourth radius of curvature.

9. The knee joint implant for preventing hyper extension of claim 8, wherein the bearing element comprises:
a fifth section having a fifth radius of curvature from an end of the fourth section and extending in the anterior direction at a fifth angle from the center of the fifth radius of curvature; and
a sixth section having a sixth radius of curvature from an end of the fifth section and extending in the anterior direction at a sixth angle from the center of the sixth radius of curvature, and
wherein points that come into contact with the condyle when the extended knee is about to be hyper-extended are formed in the fourth section and the sixth section.

10. The knee joint implant for preventing hyper extension of claim 9, wherein the fourth radius of curvature is greater than the fifth radius of curvature.

11. The knee joint implant for preventing hyper extension of claim 10, wherein the sixth radius of curvature is less than the fourth radius of curvature and greater than the fifth radius of curvature.

12. The knee joint implant for preventing hyper extension of claim 9, wherein the fourth angle is less than the fifth angle.

13. A knee joint implant for preventing hyper extension, the knee joint implant comprising:
a femur element in which an anterior surface has different radiuses of curvature in respective sections on the basis of the lowest point of a convex condyle;
a bearing element in which an anterior surface has different radiuses of curvature in respective sections on the basis of the lowest point of a concave joint facet,
wherein when an extended knee is about to be hyper-extended, the condyle of the femur element slides in an anterior direction along the joint facet of the bearing element, and then comes into contact with the joint facet at a plurality of points.

14. The knee joint implant for preventing hyper extension of claim 13, wherein the femur element comprises a first section having a first radius of curvature from the lowest point of the condyle and extending in the anterior direction at a first angle from the center of the first radius of curvature, and
wherein the bearing element comprises a fourth section having a fourth radius of curvature from the lowest point of the joint facet and extending in the anterior direction at a fourth angle from the center of the fourth radius of curvature.

15. The knee joint implant for preventing hyper extension of claim 14, wherein the femur element comprises:
a second section having a second radius of curvature from an end of the first section and extending in the anterior direction at a second angle from the center of the second radius of curvature; and
a third section having a third radius of curvature from an end of the second section and extending in the anterior direction at a third angle from the center of the third radius of curvature,
wherein the bearing element comprises:
a fifth section having a fifth radius of curvature from an end of the fourth section and extending in the anterior direction at a fifth angle from the center of the fifth radius of curvature; and
a sixth section having a sixth radius of curvature from an end of the fifth section and extending in the anterior direction at a sixth angle from the center of the sixth radius of curvature, and
wherein when the extended knee is about to be hyper-extended, the first section and the fourth section, and the third section and the sixth section form contact points, respectively.

16. The knee joint implant for preventing hyper extension of claim 15, wherein the first radius of curvature is less than the fourth radius of curvature,
wherein the second radius of curvature is less than the fifth radius of curvature, and
wherein the third radius of curvature is less than the sixth radius of curvature.

17. The knee joint implant for preventing hyper extension of claim 14, wherein an area of the first section is less than an area of the fourth section.
